# FASCICULE DE BREVET EUROPEEN

(11) **EP 2 205 549 B1**
(45) Date de publication et mention de la délivrance du brevet: **16.05.2012**
(21) Numéro de dépôt: 08843975.7
(22) Date de dépôt: 21.10.2008
(51) Int. Cl.: C07C 67/20, C07C 67/22, C07C 69/34, C07C 69/44

(54) **PROCÉDÉ AMÉLIORE DE FABRICATION DE DIESTERS**
VERBESSERTES VERFAHREN ZUR HERSTELLUNG VON DIESTERN
IMPROVED METHOD FOR PRODUCING DIESTERS

(30) Priorité: 31.10.2007 FR 0707666
(43) Date de publication de la demande: 14.07.2010
(73) Titulaire: Rhodia Opérations, 93306 Aubervilliers (FR)
(72) Inventeur: JACQUOT, Roland, F-69340 Francheville (FR); LECONTE, Philippe, F-68150 Ribeauville (FR)
(74) Mandataire: Jacobson, Claude
(86) Numéro de dépôt international: PCT/EP2008/064218
(87) Numéro de publication internationale: WO 2009/056477

(56) Documents cités:
- WO-A-2007/101929
- WO-A-2008/009792
- DE-C- 730 518
- XUE CUIHUA ET AL: "TRANSFORMATION OF AMIDES INTO ESTERS BY THE USE OF CHLOROTRIMETHYLSILANE" JOURNAL OF THE CHINESE CHEMICAL SOCIETY, vol. 51, no. 2, 1 janvier 2004 (2004-01-01), pages 359-362, XP008077858 ISSN: 0009-4536
- BOGERT M T: "ON THE PRODUCTION OF THE IMIDES OF SUCCINIC AND GLUTARIC ACIDS BY THE PARTIAL HYDRATION OF THE CORRESPONDING NITRILES" JOURNAL OF THE AMERICAN CHEMICAL SOCIETY, vol. 24, 1 janvier 1902 (1902-01-01), pages 20-25, XP002484940 ISSN: 0002-7863
- LAECKMANN D. ET AL: "Synthesis and biological evaluation of aroylguanidines related to amiloride as inhibitors of the human platelet Na<+>/H<+> exchanger" BIOORGANIC & MEDICINAL CHEMISTRY,, vol. 10, no. 6, 1 janvier 2002 (2002-01-01), pages 1793-1804, XP002429666 ISSN: 0968-0896
- BIZILJ, S: "The self-reactions of 1-(methoxycarbonyl)-1-methylethyl and higher ester radicals: combination vs. disproportionation and oligomeric products from secondary reactions" AUSTRALIAN JOURNAL OF CHEMISTRY, vol. 38, no. 11, 1985, pages 1657-1673, XP008093241 ISSN: 0004-9425

## Description

La présente invention concerne un procédé de fabrication de diesters à partir de composés dinitriles.

Elle se rapporte plus particulièrement à un procédé de fabrication de composés diesters à partir de composés dinitriles mettant en oeuvre une hydrolyse de composés dinitriles.

Elle concerne encore plus particulièrement un procédé de fabrication de diesters à partir de composés dinitriles ramifiés tels que le méthylglutaronitrile ou les composés dinitriles ramifiés obtenus comme sous produits dans le procédé de fabrication de l'adiponitrile par hydrocyanation du butadiène.

Les solvants oxygénés à base de diesters sont de plus en plus utilisés en remplacement d'autres solvants hydrocarbonés, chlorés ou oxygénés plus agressifs pour l'environnement.

En effet, les solvants diesters tels que ceux commercialisés sous le nom connu de Rhodiasolv® RDPE obtenus à partir d'un mélange d'acide adipique, acide glutarique et acide succinique présentent l'avantage d'avoir un profil toxicologique très favorable et sont biodégradables et facilement recyclables. Il a également été proposé dans la demande de brevet WO2007/101929 des composés diesters obtenus à partir de composés ramifiés et plus particulièrement d'un mélange de méthyl-glutaronitrile, éthyl-succinonitrile et adiponitrile.

Dans cette demande de brevet un procédé de fabrication a été décrit, consistant à faire réagir les composés dinitriles avec un alcool en présence d'un acide minéral suivi par une hydrolyse. Ce procédé est connu sous le nom de réaction de PINNER. Toutefois, un sel d'ammonium est obtenu comme sous-produit dans ce procédé.

Un des buts de la présente invention est de proposer un procédé de fabrication de diesters à partir de composés dinitriles ne présentant pas les inconvénients des procédés de l'art antérieur et notamment ne générant pas d'effluents ou sous-produits importants et éventuellement nocifs pour l'environnement.

A cet effet, l'invention a pour objet un procédé de fabrication d'au moins un composé diester comprenant les étapes suivantes:
a) préparation d'au moins un composé imide de forme générale (I) suivante: 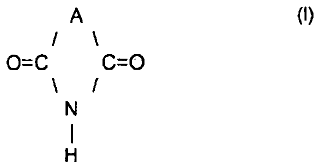 où A représente un radical divalent hydrocarboné comprenant de 2 à 12 atomes de carbones, linéaire ou ramifié
   par hydrolyse en présence d'eau d'au moins un composé dinitrile de formule générale (III) suivante :

   NC-A-CN (III)
b) puis réaction entre le composé imide avec au moins un alcool de formule générale (II) suivante:

   R - OH (II)

   où R représente un radical hydrocarboné pouvant comprendre des hétéroatomes, linéaires ou ramifiés, aliphatique, cycloaliphatique, aromatique ou arylalkyl comprenant de 1 à 20 atomes de carbone,
   de manière à obtenir un produit de réaction comprenant au moins un composé diester de formule générale (IV) suivante, et éventuellement des sous-produits de formule(s) différente(s)

   R-OOC-A-COO-R (IV)

   caractérisé en ce que:
   - l'étape a) est mise en oeuvre en phase vapeur en présence d'un catalyseur acide solide et
   - l'étape b) est mise en oeuvre en présence d'au moins un catalyseur différent de celui mis en oeuvre lors de l'étape a).

La présente specification décrit les produits, dont des compositions de matière, susceptibles d'être obtenus, ou directement obtenus, par ce procédé. La présente specification décrit aussi l'utilisation de ces produits ou compositions de matière, notamment à titre de solvants, co-solvants, inhibiteurs de cristallisation, agents de nettoyage et/ou de dégraissage, agents décapant.

### Définitions

Dans la description, on désigne par «catalyseur», le matériau catalyseur sous sa forme native ou en mélange avec une matrice ou un support préparé selon des techniques connues de l'homme du métier.

Par catalyseur acide, on entend un catalyseur acide au sens de Lewis, tel que défini dans la littérature, notamment par Jerry MARCH, Advanced Organic Chemistry, 3ème édition, John Wiley and Sons, 1985, pp. 227 et suivantes, ou un catalyseur identifié comme tel dans la présente demande.

Par catalyseur basique, on entend un catalyseur basique au sens de Lewis, tel que défini dans la littérature, notamment par Jerry MARCH, Advanced Organic Chemistry, 3ème édition, John Wiley and Sons, 1985, pp. 227 et suivantes, ou un catalyseur identifié comme tel dans la présente demande.

Par substantiellement sans catalyseur, on entend:
- que n'est pas mis en oeuvre un lit fixe de catalyseur, et
- que ne sont pas mis en oeuvre des catalyseurs hors lit fixe en quantité en poids, support exclu si présent, par rapport aux réactifs, supérieure à 1 %, de préférence à 0,5%, de préférence à 0.1%, de préférence à 0,01%.

Dans la présente demande une composition de matière désigne un mélange de plusieurs composés, par exemple un produit de réaction comprenant plusieurs composés. Il peut s'agir notamment de produits issus de réactifs en mélanges, présentant un même type de fonctions réactives. Une composition de matière comprend de préférence au moins 50% en poids de composés répondant à une même formule chimique (formule précise, ou formule générale, ou formule moyenne), de préférence au moins 75%, de préférence au moins 90%, de préférence au moins 99%.

L'étape a) peut être considérée comme une étape d'hydrolyse cyclisant. Dans la présente demande, on se réfère également à l'étape a) comme "hydrolyse cyclisante".

### Conditions opératoires

L'étape a) et/ou l'étape b) peuvent être mise en oeuvre en phase vapeur. L'étape b) peut être mise en oeuvre en phase liquide ou en phase vapeur. Selon un mode de réalisation les deux étapes sont mises en oeuvre en phase vapeur. Selon un autre mode de réalisation l'étape a) en mise en oeuvre en phase vapeur et l'étape b) est mise en oeuvre en phase liquide. Pour les étapes mises en oeuvre en phase vapeur le milieu réactionnel peut être mis en contact avec le catalyseur après avoir été vaporisé.

L'étape a) est mise en oeuvre en phase vapeur en présence d'un catalyseur solide.

Selon un mode de réalisation particulier,
- l'étape a) est mise en oeuvre en présence d'un catalyseur solide acide, et
- l'étape b) est mise en oeuvre en présence d'un catalyseur basique.

Ces deux modes permettent notamment d'obtenir une conversion importante, et/ou une sélectivité importante, et/ou de limiter les sous-produits indésirables, ne pouvant par exemple pas être réintroduits dans le procédé de préparation après une transformation aisée.

Des catalyseurs utiles, acides ou basiques, sont mentionnés plus bas.

L'étape a) est avantageusement mise en oeuvre à une température inférieure à 500 °C, de préférence comprise entre 250 °C et 450°C. Par ailleurs, le rapport molaire entre l'eau et le composé nitrile est avantageusement compris entre 2 et 20 et préférentiellement compris entre 4 et 8.

L'étape b) est avantageusement mise en oeuvre avec un rapport molaire entre l'alcool et le composé imide compris entre 1 et 30 et préférentiellement compris entre 5 et 20. Pour cette étape, on préfère utiliser un excès important d'alcool, et éventuellement réutiliser par la suite l'excès qui n'a pas réagi. Ceci permet en particulier d'augmenter la sélectivité.

L'étape b) est avantageusement réalisée en phase liquide à une température inférieure à 400 °C de préférence entre 100 et 300 °C, par exemple entre 150°C et 250°C, de préférence à une pression de 1 à 100 bars, notamment de 10 à 100 bars, par exemple entre 15 et 25 bars, ou entre 30 et 50 bars, de préférence la pression autogène. Selon un mode de réalisation préférentiel, l'étape b) est mise en oeuvre en phase liquide, l'alcool étant utilisé à la fois comme réactif et comme milieu solvant, en excès.

On mentionne que lors de l'étape b) il se forme de l'ammoniac. On peut l'éliminer au cours de cette étape, par exemple en le soutirant du réacteur sous forme gazeuse (dans un ciel gazeux du réacteur si l'étape b) est réalisée en phase liquide). Il peut notamment être soutiré à l'aide d'un dispositif approprié, par exemple permettant de conserve la pression constante, par exemple laissant échapper du gaz lorsque la pression dépasse une certaine valeur, et permettant le cas échéant une liquéfaction après échappement. Ce dispositif peut être séparé du réacteur par une conduite. L'élimination de l'ammoniac permet notamment de favoriser la réaction et de limiter la formation de sous-produits. L'élimination de l'ammoniaque peut être accompagnée d'une élimination simultanée d'alcool également sous forme gazeuse. On cherche de préférence à limiter l'élimination d'alcool simultanée. On peut par exemple à cet effet refroidir les gaz le long d'une conduite séparant le réacteur dispositif, de manière à liquéfier au moins une partie de l'alcool et le refluer vers le réacteur. Le(s) gaz éliminé(s) peut (peuvent) être récupéré(s) et réutilisé(s), le cas échéant après séparation de l'ammoniac et de l'alcool. Après séparation, l'alcool peut être réutilisé pour la mise en oeuvre de l'étape b).

L'étape a) et l'étape b) peuvent être mises en oeuvre en continu ou en discontinu, dans des types de réacteurs permettant éventuellement d'utiliser un catalyseur solide soit sous forme de lit fixe ou de lit fluidisé. La réaction peut être conduite sous pression atmosphérique ou sous pression plus élevée par exemple sous une pression pouvant aller jusqu'à 100 bars, de préférence jusqu'à 30 bars. Il peut s'agir de la pression autogène du milieu réactionnel à la température ou les étapes sont opérées.

Le composé diester après condensation est extrait du milieu réactionnel par les techniques usuelles de séparation et purification des composés organiques telles que distillation, extraction liquide/liquide, par exemple.

On note que le composé imide obtenu par hydrolyse du composé dinitrile peut être avantageusement séparé du milieu réactionnel et purifié par les techniques usuelles. Toutefois, il est également possible d'utiliser directement comme réactif dans l'étape de réaction avec un alcool, le milieu réactionnel obtenu après l'étape d'hydrolyse, sans séparation ni purification.

On note en particulier que le procédé peut comprendre l'étape suivante c), après l'étape b):
étape c): chauffage du produit de réaction de l'étape b) et distillation de manière à récupérer le composé diester.

L'étape b) quand elle est réalisée substantiellement sans catalyseur, ou en présence d'un catalyseur basique peut mener à des sous produits qu'il est possible de retransformer aisément en imide, notamment par chauffage, par exemple lors d'une distillation. On a trouvé que la mise en oeuvre d'un catalyseur acide lors de cette étape peut favoriser la formation de sous produits non désirés et non facilement transformables. Ainsi le produit de réaction de l'étape b) peut comprendre, en particulier lorsque cette étape est mise en oeuvre substantiellement sans catalyseur ou en présence d'un catalyseur basique, un sous-produit qu'on transforme avantageusement en imide de formule (I) lors de l'étape c), et qui est re-utilisé pour la mise en oeuvre de l'étape b).

Le composé dinitrile est de préférence choisi dans le groupe comprenant le méthylglutaronitrile, l'éthylsuccinonitrile, l'adiponitrile et leurs mélanges.

L'alcool est de préférence choisi dans le groupe comprenant méthanol, propanol, isopropanol, alcool benzylique, éthanol, n-butanol, isobutanol, pentanols, cyclohexanol, hexanol, isooctanol, 2-éthyl hexanol et leurs mélanges. On peut mettre en oeuvre des mélanges d'alcools, comme de l'huile de Fusel.

### Catalyseurs acides

Le catalyseur acide est de préférence un catalyseur solide. Il peut notamment s'agir d'un catalyseur acide solide, typiquement utilisé en phase hétérogène, par exemple choisi parmi:
- les oxydes métalliques comme l'alumine, les oxydes de titane, les mélanges silice/alumine et analogues,
- les zéolithes sous forme acide,
- les argiles sous forme acide,
- les phosphates acides, comme NaH₂PO₄ ou le pyrophosphate de silicium.

Les catalyseurs acides peuvent se présenter sous différentes formes dans le procédé de l'invention: poudre, billes, concassés, extrudés en forme de granulés cylindrique creux ou pleins, de nid d'abeille, pastilles, la mise en forme pouvant éventuellement être réalisée à l'aide d'un liant produit. Ces formes peuvent être obtenues notamment par extrusion, moulage, compactage ou tout autre type de procédé connu. En pratique, sur le plan industriel, ce sont les formes de granulés, de billes ou d'extrudés qui présentent le plus d'avantages tant sur le plan de l'efficacité que sur le plan de la commodité de mise en oeuvre.

Par «zéolithe», on entend un tectosilicate cristallisé d'origine naturelle ou synthétique dont les cristaux résultent de l'assemblage tridimensionnel d'unités tétraédriques de SiO₄ et TO₄ : T représentant un élément trivalent tel que aluminium, gallium, bore, fer, de préférence, l'aluminium. Les zéolithes de type aluminosilicate sont les plus communes. Les zéolithes présentent au sein du réseau cristallin, un système de cavités reliées entre elles par des canaux d'un diamètre bien défini que l'on appelle les pores. Elles peuvent présenter un réseau de canaux monodimensionnel, bidimensionnel ou tridimensionnel. On peut mettre en oeuvre une zéolithe naturelle ou synthétique.

Comme exemples de zéolithes naturelles susceptibles d'être utilisées, on peut citer, par exemple : la chabazite, la clinoptilolite, l'érionite, la phillipsite et l'offrétite.

Conviennent également les zéolithes synthétiques. A titre illustratif de celles-ci, on peut citer celles à réseau monodimensionnel, comme la zéolithe ZSM-4, la zéolithe L, la zéolithe ZSM-12, la zéolithe ZSM-22, la zéolithe ZSM-23, la zéolithe ZSM-48. A titre d'exemples de zéolithes à réseau bidimensionnel mises en oeuvre préférentiellement, on peut mentionner la mordénite, la ferrierite. En ce qui concerne les zéolithes à réseau tridimensionnel, on peut nommer plus particulièrement, la zéolithe beta, la zéolithe Y, la zéolithe X, la zéolithe ZSM-5, la zéolithe ZSM-11, l'offrétite.

On peut notamment mettre en oeuvre des zéolithes qui sont sous les formes suivantes:
- la mazzite de rapport atomique Si/Al de 3,4,
- la zéolithe L de rapport atomique Si/Al de 1,5 à 3,5,
- la mordénite de rapport atomique Si/Al de 5 à 150, de préférence, de 10 à 100 et encore plus préférentiellement de 10 à 50,
- la ferrierite de rapport atomique Si/Al de 3 à 10,
- l'offrétite de rapport atomique Si/Al de 4 à 8,5,
- les zéolithes beta de rapport atomique Si/Al de 10 à 100, de préférence, 12 à 50,
- les zéolithes Y en particulier les zéolithes obtenues après traitement de désalumination (par exemple hydrotraitement, lavage à l'aide d'acide chlorhydrique ou traitement par SiCl₄) et l'on peut citer plus particulièrement les zéolithes US-Y de rapport atomique Si/Al supérieur à 3, de préférence compris entre 6 et 60 ;
- la zéolithe X de type faujasite de rapport atomique Si/Al de 0,7 à 1,5,
- les zéolithes ZSM-5 ou silicalite d'aluminium de rapport atomique Si/Al de 10 à 500,
- la zéolithe ZSM-11 de rapport atomique Si/Al de 5 à 30.

Parmi toutes ces zéolithes, on fait appel préférentiellement dans le procédé de l'invention aux zéolites US-Y.

La zéolithe mise en oeuvre est sous forme acide. On effectue si nécessaire un traitement qui la rend acide. A cet effet, on fait appel aux traitements classiques. A des fins de clarté, le caractère acide des zéolithes mises en oeuvre dans les exemples ci-après est signalé par le préfixe H.

Par ailleurs, on peut désactiver la surface externe des zéolithes employées à titre de catalyseur. Ce type de traitement est bien connu de l'homme du métier. Il peut notamment consister en une désalumination à la vapeur d'eau ou par un traitement acide ou en une silylation.

Les zéolithes pouvant être mises en oeuvre, sont des produits connus décrits dans la littérature [cf. Atlas of zeolites structure types by W. M. Meier and D. H. Olson published by the Structure Commission of the International Zeolite Association (1992)].

La zéolithe constitue la phase catalytique. Elle peut être utilisée seule ou en mélange avec une matrice minérale. Dans le cas particulier où le catalyseur est utilisé en mélange avec une matrice, cette matrice peut être choisie parmi les oxydes de métaux, tels que les oxydes d'aluminium, de silicium et/ou de zirconium, ou encore parmi les argiles et plus particulièrement, le kaolin, le talc ou la montmorillonite. Dans un tel catalyseur, la teneur en phase active peut représenter de 5 à 100 % du poids du catalyseur.

Selon un mode particulier de l'invention, la zéolithe utilisée est une zéolite US-Y sous forme acide et de rapport Si/Al supérieur à 3 et de préférence de 10 à 50.

Les argiles convenables pouvant être mises en oeuvre à titre de catalyseur acide peuvent notamment être des phyllosilicates qui sont classés par groupes selon leur nature et leurs propriétés physico-chimiques, groupes parmi lesquels on peut citer les kaolins, les serpentines, les smectites ou montmorillonites, les illites ou micas, les glauconites, les chlorites ou vermiculites, les attapulgites ou sépiolites, les argiles à couches mixtes, les allophanes ou imogolites et les argiles à haute teneur en alumine.

Certaines argiles possèdent une structure lamellaire à réseau expansible. Elles présentent la particularité d'adsorber divers solvants, notamment l'eau, entre les feuillets qui les composent, ce qui provoque un gonflement du solide par suite de l'affaiblissement des liaisons électrostatiques entre les feuillets. Ces argiles appartiennent essentiellement au groupe des smectites (ou encore groupe de la montmorillonite) et pour certaines d'entre elles au groupe des vermiculites.

Leur structure est composée de feuillets « élémentaires » à trois couches : deux couches simples de tétraèdres SiO₄ dans lesquelles une partie du silicium peut être remplacée par d'autres cations en position tétraédrique tels que Al³⁺ ou éventuellement Fe³⁺, et entre ces deux couches de tétraèdres, une couche d'octaèdres d'oxygènes au centre desquels se situent des cations métalliques tels que Al³⁺, Fe³⁺, Mg²⁺. Cette couche octaédrique est constituée d'un empilement compact d'oxygènes provenant soit des sommets des tétraèdres précédents soit de groupes hydroxyles OH. Le réseau hexagonal compact de ces oxygènes contient 6 cavités octaédriques.

Lorsque les cations métalliques occupent 4 de ces cavités (2 cavités sur 3 comme dans le cas de l'aluminium par exemple), la couche est dite dioctaédrique; lorsqu'ils occupent toutes les cavités (3 cavités sur 3 comme dans le cas du magnésium par exemple), la couche est dite trioctaédrique.

Les feuillets élémentaires de ces argiles sont porteurs de charges négatives qui sont compensées par la présence de cations échangeables, alcalins tels que Li⁺, Na⁺, K⁺, alcalino-terreux tels que Mg²⁺ Ca²⁺ et éventuellement l'ion hydronium H₃O⁺. Les smectites ont des densités de charge sur les feuillets inférieures à celles des argiles du type vermiculites : environ 0,66 charges par maille élémentaire contre 1 à 1,4 charges par maille élémentaire pour les vermiculites.

Les cations de compensation sont essentiellement le sodium et le calcium dans les smectites, le magnésium et le calcium dans les vermiculites. Du point de vue des densités de charges, smectites et vermiculites sont intermédiaires entre le talc et la pyrophyllite d'une part, dont les feuillets sont neutres et les micas d'autre part, caractérisés par une densité de charges importante sur les feuillets (environ 2 par maille élémentaire) compensée généralement par des ions K⁺.

Les cations interfoliaires des smectites et des vermiculites peuvent être assez facilement remplacés par échange ionique par d'autres cations tels, par exemple, des ions ammonium ou des ions de métaux alcalino-terreux ou de métaux de terres rares.

Les propriétés de gonflement des argiles dépendent de divers facteurs dont la densité de charge et la nature du cation de compensation. Ainsi les smectites dont la densité de charge est plus faible que celle des vermiculites présentent des propriétés gonflantes nettement supérieures à celles de ces dernières, et constituent donc une classe très intéressante de solides. La distance répétitive ou espacement basal représente la distance la plus courte séparant deux motifs cristallographiquement identiques situés dans deux feuillets adjacents. L'espacement basal des smectites peut ainsi atteindre par gonflement des valeurs allant de 1 nm environ à plus de 2 nm.

Parmi les silicates phylliteux « gonflants » du type smectites, on peut citer les principaux solides suivants de formule générale:

(M₁ⁿ⁺)_{x/n} (M₂)₂^{VI} (M₃)₄^{IV} O₁₀ (OH)₂

où M₁ est le cation interfoliaire
M₂ est le métal en position octaédrique
M₃ est le métal en position tétraédrique
x est le nombre de charges apportées par le cation M₁
Les smectites dioctaédriques
montmorillonite (H, Na, Ca_{1/2})ₓ (MgₓAl₂₋ₓ)^{VI} Si₄^{IV} O₁₀ (OH)₂
beidellite (H, Na, Ca_{1/2})ₓ Al₂^{VI} (AlₓSi₄₋ₓ)^{IV} O₁₀ (OH)₂
nontrolite (H, Na, Ca_{1/2}...)ₓ (Fe, Al)₂^{VI} (AlₓSi₄₋ₓ)^{IV} O₁₀ (OH)₂
Les smectites trioctaédriques
hectorite Naₓ (LiₓMg₃₋ₓ)^{VI} Si₄^{IV} O₁₀ (OH)₂
saponite Naₓ Mg₃^{VI} (AlₓSi₄₋ₓ)^{IV} O₁₀ (OH)₂
stevensite Na₂ₓ Mg₃₋ₓ^{VI} Si₄^{IV} O₁₀ (OH)₂

Après adsorption à saturation d'eau ou d'un solvant polaire organique dans une smectite, l'espacement interfoliaire (entre deux feuillets) est maximal. Il peut atteindre une valeur voisine de 1 nm.

Ces solides sont donc potentiellement intéressants en catalyse car leur surface spécifique et leur acidité potentielles sont élevées.

Selon un mode particulier de l'invention, l'argile qui constitue le catalyseur acide est une smectite. Plus préférentiellement l'argile est la montmorillonite.

Certaines argiles ont malheureusement l'inconvénient de perdre leur caractère expansé par chauffage à 100°C et de ce fait de ne pas conserver l'augmentation de surface spécifique résultant de leur expansion. C'est le cas notamment des smectites. Différentes méthodes ont été décrites dans l'art antérieur pour introduire entre les feuillets des smectites des piliers ou ponts pour obtenir des smectites pontées qui conservent un espacement interfoliaire élevé après avoir été soumises à un traitement thermique.

Une méthode consistant à introduire des ponts constitués par des oligomères d'un hydroxyde d'un métal, notamment d'hydroxyde d'aluminium, a été décrite par LAHAV, SHAMI et SHABTAI dans Clays and Clays Mineral, vol.26 (n°2), p. 107-115 (1978) et dans le brevet français 2.394.324. La formation de ponts constitués d'oligomères d'hydroxydes mixtes de silicium et de bore, est décrite dans le brevet US 4.248.739. Une technique de pontage des smectites, par dialyse, à l'aide d'hydroxydes d'aluminium, de chrome, de zirconium et titane etc...est revendiquée dans le brevet EP 0.073.718.

Ces méthodes consistent dans leur principe à mettre l'argile au contact d'une solution contenant des espèces ioniques plus ou moins oligomérisées du type hydroxy-aluminique (dans le cas de l'aluminium). Cette opération est réalisée généralement en solution peu concentrée, à température inférieure à 80°C et si possible en l'absence de trouble constitué par un début de précipitation de l'hydroxyde métallique. Les concentrations de l'ion métallique et de l'argile doivent être optimisées pour qu'il y ait formation de suffisamment de piliers solides et que la porosité de l'argile ne soit pas fortement diminuée par l'insertion d'une trop grande quantité d'oxyde métallique.

Lorsque les ions interfoliaires alcalins ou alcalino-terreux sont remplacés par des protons soit directement à l'aide d'une solution très diluée, soit de préférence par échange avec un sel d'ammonium suivi d'une calcination entre 300 et 700°C, les smectites pontées acquièrent une acidité forte quoique inférieure globalement à celles des zéolithes classiques de type Y ou mordenite par exemple.

Selon une variante particulière de l'invention, le catalyseur peut comporter, outre une argile, un ou plusieurs autres composés métalliques, souvent appelés dopants, tels que par exemple des composés de chrome, de titane, de molybdène, de tungstène, de fer, de zinc. Parmi ces dopants les composés de chrome et/ou de fer et/ou de titane sont considérés comme les plus avantageux. Ces dopants représentent habituellement, en poids par poids d'argile, de 0 % à 10 % et de préférence de 0 % à 5 %. Par composé métallique ici on entend aussi bien l'élément métal que l'ion métallique ou toute combinaison comprenant l'élément métal.

Une autre classe de catalyseur acide consiste dans un catalyseur particulaire obtenu par mise en forme d'au moins un oxyde minéral simple ou mixte d'au moins un élément choisi dans le groupe consistant en le silicium, l'aluminium, le titane, le zirconium, le vanadium, le niobium, le tantale, le tungstène, le molybdène, le fer. Ces oxydes peuvent se trouver sous une forme amorphe ou cristalline. On cite en particulier le dioxyde de titane, de préférence sous forme anatase. Il peuvent notamment comprendre un support.

Le catalyseur particulaire peut présenter une macroporosité caractérisée par un volume poreux correspondant aux pores de diamètre supérieur à 500 Å, supérieur ou égal à 5ml/100g. Cette macroporosité est formée avantageusement pendant le procédé de mise en forme des particules par des techniques décrites ci-dessous ou comme, par exemple, l'addition de porogène.

Il peut s'agir tout d'abord de billes d'oxydes minéraux issues d'une mise en forme par oil-drop (ou coagulation en gouttes). Ce type de billes peut par exemple être préparé par un procédé similaire à celui décrit pour la formation de billes d'alumine dans les brevets EP-A-0 015 801 ou EP-A-0 097 539. Le contrôle de la porosité peut être réalisé en particulier, selon le procédé décrit dans le brevet EP-A-0 097 539, par coagulation en gouttes d'une suspension, d'une dispersion aqueuse d'oxyde minéral. Les billes peuvent être également obtenues par le procédé d'agglomération dans un drageoir ou tambour tournant.

Il peut aussi s'agir d'extrudés d'oxydes minéraux. Ceux-ci peuvent être obtenus par malaxage, puis extrusion d'une matière à base de l'oxyde minéral. Le contrôle de la porosité de ces extrudés peut être réalisé par le choix de l'oxyde mis en oeuvre et par les conditions de préparation de cet oxyde ou par les conditions de malaxage de cet oxyde avant extrusion. L'oxyde minéral peut ainsi être mélangé lors du malaxage à des porogènes. A titre d'exemple, les extrudés peuvent être préparés par le procédé décrit dans le brevet US 3 856 708.

De manière semblable, des billes à porosité contrôlée peuvent être obtenues par addition de porogène et agglomération dans un bol tournant ou drageoir ou par le procédé 'Oil-drop'.

Selon un mode de réalisation particulier, les particules de catalyseur présentent une surface spécifique supérieure à 10 m²/g et un volume poreux égal ou supérieur à 10 ml/100 g, le volume poreux correspondant aux pores de diamètre supérieur à 500 Å étant supérieur ou égal à 10 ml/100 g.

Selon autre mode de réalisation particulier, les particules de catalyseur présentent une surface spécifique supérieure à 50 m²/g.

Avantageusement, elles présentent un volume poreux total supérieur ou égal à 15 ml/100g avec un volume poreux correspondant aux pores de diamètre supérieur à 200 Å, supérieur ou égal à 15 ml/100g, de préférence supérieur ou égal à 20ml/100g.

Ces catalyseurs particulaires peuvent également comprendre au moins un élément choisi dans la liste consistant en le silicium, le titane, le zirconium, le vanadium, le niobium, le tantale, le tungstène, le molybdène, le fer, ou être obtenu par dépôt et/ou adsorption sur le support d'au moins un composé oxygéné d'au moins un élément choisi dans le groupe consistant en les éléments appartenant aux groupes 1 à 16 de la classification universelle des éléments (nouvelle classification). Ces éléments ou composés sont déposés, adsorbés ou co-malaxés, sur ou avec le catalyseur particulaire.

Dans le mode opératoire comprenant un catalyseur particulaire poreux supportant des composés oxygénés d'éléments, ces éléments sont avantageusement choisis dans la liste comprenant le silicium, le titane, le zirconium, le vanadium, le niobium, le tantale, le tungstène, le molybdène, le phosphore, le bore, le fer, les alcalins, les alcalino-terreux, les terres rares. Le composé oxygéné est avantageusement un oxyde simple ou mixte de un ou plusieurs des éléments cités ci-dessus.

Dans ce mode de réalisation, le catalyseur poreux est de préférence un oxyde d'aluminium. Avantageusement, cette alumine présente les caractéristiques de surface spécifique et distribution de pores définis précédemment.

La concentration pondérale en composé oxygéné supporté sur un support poreux est avantageusement comprise entre 1000 ppm et 30% exprimée en masse d'élément du composé oxygéné par rapport à la masse totale du catalyseur. Cette concentration est plus préférentiellement comprise entre 0,5 % et 15 % en poids.

Quand les supports poreux correspondent à des alumines conformes à l'invention, celles-ci sont obtenues généralement par déshydratation de gibbsite, bayerite, nordstandite ou de leurs différents mélanges. Les différents procédés de préparation des alumines sont décrits dans l'encyclopédie KIRK-OTHMER, volume 2, pages 291 - 297.

On peut préparer les alumines mises en oeuvre dans le présent procédé par mise en contact d'une alumine hydratée, sous forme finement divisée, avec un courant de gaz chaud à une température comprise entre 400°C et 1000°C, puis maintien du contact entre l'hydrate et les gaz pendant une durée allant d'une fraction de seconde jusqu'à 10 secondes et enfin séparation de l'alumine partiellement déshydratée et des gaz chauds. On peut notamment se référer au procédé décrit dans le brevet américain US2915365.

On peut également procéder à l'autoclavage d'agglomérés d'alumines obtenues précédemment, en milieu aqueux, éventuellement en présence d'acide, à une température supérieure à 100°C et de préférence comprise entre 150°C et 250°C, pendant une durée de préférence comprise entre 1 et 20 heures, puis à leur séchage et leur calcination.

La température de calcination est réglée de telle façon que l'on obtienne des surfaces spécifiques et des volumes poreux situés dans les zones de valeurs indiquées précédemment.

Dans un mode de réalisation particulier, on met un oeuvre un catalyseur, un catalyseur particulaire poreux à base de dioxyde de titane, de préférence sous forme anatase, supportant éventuellement un composé oxygéné choisi dans la liste consistant en le silicium, l'aluminium, le zirconium, le vanadium, le niobium, le tantale, le tungstène, le molybdène, le fer, ou être obtenu par dépôt et/ou adsorption sur le support d'au moins un composé oxygéné d'au moins un élément choisi dans le groupe consistant en les éléments appartenant aux groupes 1 à 16 de la classification universelle des éléments (nouvelle classification). Ces éléments ou composés sont déposés, adsorbés ou co-malaxés, sur ou avec le catalyseur particulaire. La teneur en dioxyde de titane peut par exemple être de 10 à 80% en poids, le reste étant par exemple de la silice ou de l'alumine.

Les catalyseurs de l'invention ont avantageusement une surface spécifique supérieure à 50 m²/g.

En outre, ils présentent avantageusement des pores de diamètre supérieur à 0,1 µm, le volume poreux apporté par ces pores étant supérieur ou égal à 5ml/100g, avantageusement supérieur ou égal à 10ml/100g.

Dans un mode de réalisation préféré de l'invention, ces catalyseurs comprennent également des pores de diamètre égal ou supérieur à 0,5 µm, le volume poreux correspondant étant égal ou supérieur à 5 ml/100 g, de préférence supérieur ou égal à 10 ml/100 g.

Ce volume poreux généré par les pores de diamètre supérieur à 500 Å, de préférence supérieur à 0,1 µm et avantageusement supérieur à 0,5 µm permet d'obtenir des catalyseurs à durée de cycle élevée.

Selon l'invention, les catalyseurs comprenant des composés oxygénés supportés par un catalyseur poreux sont obtenus, généralement par imprégnation du catalyseur, notamment de l'alumine, par une solution d'un sel ou composés des éléments cités précédemment, puis séchés et calcinés à une température égale ou supérieure à 400°C, pour transformer éventuellement et avantageusement lesdits composés ou sels en composés oxygénés, de préférence en oxydes. Les oxydes sont déposés à la surface des pores du catalyseur poreux.

Dans un autre mode de réalisation, les composés d'éléments peuvent être ajoutés dans le matériau constituant le catalyseur poreux avant sa mise en forme ou au cours du processus de mise en forme.

La calcination des catalyseurs imprégnés est de préférence réalisée sous atmosphère oxydante telle que l'air.

On peut notamment mettre en oeuvre, à titre de catalyseur acide, un phosphate acide, généralement métallique, de formule générale :

(PO₄)ₙ Hₕ M, (Imp)ₚ

dans laquelle :
- M représente un élément divalent, trivalent, tétravalent ou pentavalent choisi dans les groupes 2a, 3b, 4b ,5b, 6b, 7b, 8, 2b, 3a, 4a et 5a de la classification périodique des éléments ou un mélange de plusieurs de ces éléments ou M=O,
- Imp représente un composé d'imprégnation basique constitué d'un métal alcalin ou alcalino-terreux, ou de mélanges de plusieurs de ces métaux, associé à un contre-anion pour assurer la neutralité électrique,
- n représente 1, 2 ou 3,
- h représente 0, 1 ou 2,
- p représente un nombre compris entre 0 et 1/3 et correspond à un rapport molaire entre l'imprégnant Imp et l'imprégné (PO₄)ₙ Hₕ M.

Parmi les métaux des groupes 2a, 3b, 4b ,5b, 6b, 7b, 8, 2b, 3a, 4a et 5a de la classification périodique des éléments, on peut citer notamment le béryllium, le magnésium, le calcium, le strontium, le baryum, l'aluminium, le bore, le gallium, l'indium, l'yttrium, les lanthanides tels que le lanthane, le cérium, le praséodyme, le néodyme, le samarium, l'europium, le gadolinium, le terbium, le dysprosium, l'holmium, l'erbium, le thulium, l'ytterbium et le lutécium, le zirconium, le titane, le vanadium, le niobium, le fer, le germanium, l'étain, le bismuth.

Parmi les phosphates de lanthanides, on peut distinguer une première famille qui regroupe les orhophosphates de terres rares légères, également dénommées terres rares cériques, incluant le lanthane, le cérium, le praséodyme, le néodyme, le samarium et l'europium. Ces orthophosphates sont dimorphiques. Ils présentent une structure hexagonale et évoluent vers une structure monoclinique, lorsqu'ils sont chauffés à une température de 600 à 800°C.

Une deuxième famille de phosphates de lanthanides regroupe les orthophosphates de gadolinium, de terbium et de dysprosium. Ces orthophosphates présentent la même structure que les orthophosphates de terres rares cériques, mais présentent en plus une troisième phase cristalline de structure quadratique à haute température (vers 1700°C).

Une troisième famille de phosphates de lanthanides regroupe les orthophosphates de terres rares lourdes, appelées également terres rares yttriques, incluant l'yttrium, l'holmium, l'erbium, le thulium, l'ytterbium et le lutécium. Ces composés cristallisent uniquement sous la forme quadratique.

Parmi les différentes familles d'orthophosphates de terres rares précitées, on fait appel préférentiellement aux orthophosphates de terres rares cériques.

On peut mettre en oeuvre des phosphates métalliques de formule précédente qui sont des mélanges de phosphates de plusieurs des métaux indiqués précédemment ou des phosphates mixtes de plusieurs des métaux indiqués précédemment ou encore des phosphates mixtes contenant un ou plusieurs des métaux indiqués précédemment et un ou plusieurs autres métaux tels que les métaux alcalins ou alcalino-terreux.

Les contre-anions entrant dans la formule du composé d'imprégnation Imp sont basiques. On peut notamment utiliser les ions hydroxyde, phosphate, hydrogénophosphate, dihydrogénophosphate, chlorure, fluorure, nitrate, benzoate, oxalate, sans que ces citations soient limitatives.

Le rapport molaire p est de préférence compris entre 0,02 et 0,2.

Si l'on se réfère aux techniques générales de préparation de phosphates (telles que décrites notamment dans "PASCAL P. Nouveau traité de chimie minérale" tome X (1956), pages 821-823 et dans "GMELINS Handbuch der anorganischen Chemie" (8ème édition) volume 16 (C), pages 202-206 (1965), on peut distinguer deux voies principales d'accès aux phosphates. D'une part, la précipitation d'un sel soluble du métal (chlorure, nitrate) par l'hydrogénophosphate d'ammonium ou l'acide phosphorique. D'autre part, la dissolution de l'oxyde ou du carbonate du métal (insolubles) avec de l'acide phosphorique, généralement à chaud, suivie d'une précipitation.

Les phosphates précipités obtenus selon l'une des voies indiquées peuvent être séchés, traités par une base organique (telle que l'ammoniaque) ou minérale (telle qu'un hydroxyde de métal alcalin) et être soumis à une calcination, ces trois opérations pouvant être réalisées dans l'ordre indiqué ou dans un ordre différent.

Les phosphates métalliques de formule précédente pour lesquels le symbole p est supérieur à 0, peuvent être préparés par imprégnation du composé (PO₄)ₙ Hₙ M préparé selon l'une des techniques décrites précédemment, avec une solution ou une suspension de Imp dans un solvant volatil, tel que l'eau de préférence. Les résultats sont d'autant meilleurs que Imp est plus soluble et que le composé (PO₄)ₙ Hₕ M est plus fraîchement fabriqué.

Ainsi un procédé avantageux de préparation de ces phosphates consiste :
a) à réaliser la synthèse du composé (PO₄)ₙ Hₕ M ; puis de préférence sans séparer (PO₄)ₙ Hₕ M du milieu réactionnel ;
b) à introduire l'imprégnant Imp dans le milieu réactionnel ;
c) à séparer l'éventuel liquide résiduel du solide réactionnel ;
d) à sécher et éventuellement à calciner.

Les performances de ces catalyseurs et notamment leur résistance à la désactivation peuvent être encore améliorées par une calcination. La température de calcination sera avantageusement comprise entre 300°C et 1000°C et de préférence entre 400°C et 900°C. La durée de la calcination peut varier dans de larges limites. A titre indicatif, elle se situe généralement entre 1 heure et 24 heures.

Parmi les catalyseurs pouvant être utilisés, on peut citer plus particulièrement le phosphate de lanthane, le phosphate de lanthane calciné, le phosphate de lanthane associé à un dérivé du césium, du rubidium ou du potassium, le phosphate de cérium calciné, le phosphate de cérium associé à un composé du césium, du rubidium ou du potassium, le phosphate de samarium associé à un composé du césium, du rubidium ou du potassium, le phosphate d'aluminium, le phosphate d'aluminium associé à un composé du césium, du rubidium ou du potassium, le phosphate de niobium calciné, le phosphate de niobium associé à un composé du césium, du rubidium ou du potassium, l'hydrogénophosphate de zirconium calciné, l'hydrogénophosphate de zirconium associé à un composé du césium, du rubidium ou du potassium.

Les orthophosphates décrits ci-dessus peuvent être utilisés en mélange avec de l'acide phosphorique (H₃PO₄).

On peut également utiliser comme catalyseur des pyrophosphates de terres rare, notamment de lanthane, seuls ou en mélange avec les orthophosphates décrits ci-dessus. De tels catalyseurs sont décrits dans le brevet européen EP1066255.

### Catalyseurs basiques

Le catalyseur basique peut être solide ou non. Il peut être mis en oeuvre sous forme hétérogène ou non, notamment lors de l'étape b). Il peut notamment être mis en oeuvre sous forme solubilisée dans le milieu réactionnel, notamment lors de l'étape b).

Selon un premier mode de réalisation, on peut mettre en oeuvre, à titre de catalyseur basique, un sel organique comprenant un anion basique. Conviennent notamment les sels alcalins ou alcalino-terreux de composés comprenant un groupe sulfate, sulfonate, phosphate ou phosphonate, ou de composés organiques comprenant un groupe carboxylate ou alcoolate (ou "alkylate"). On cite notamment les alcoolates de potassium, sodium ou de lithium, notamment l'éthanolate de sodium ou l'éthanolate de lithium.

Selon un deuxième mode de réalisation, on peut mettre en oeuvre, à titre de catalyseur basique, une base minérale. Il peut s'agir d'une base minérale azotée ou non azotée.

Les bases minérales autres que les bases azotées ont pour avantage d'être d'un coût plus modéré et d'être moins nuisibles sur le plan de l'environnement. Enfin, on se préserve de toute réaction secondaire susceptible d'être observée avec les amines primaires ou secondaires par exemple.

Conviennent notamment les sels alcalins hydrosolubles de type hydroxydes, carbonates inorganique, phosphates inorganiques. A titre illustratif de ces bases, on peut notamment citer les hydroxydes tels NaOH, KOH, LiOH et les sels de bases fortes avec un acide faible tels K₂CO₃ et Na₂CO₃, K₃PO₄, Li₃PO₄.

Selon un troisième mode de réalisation, on peut mettre en oeuvre, à titre de catalyseur basique, un catalyseur basique hétérogène, solide. Dans ce cas particulier, la base utilisée peut être un catalyseur hétérogène à base d'hydroxydes et/ou d'oxydes de d'alcalins, d'alcalino-terreux et/ou de lanthanides. Il peut notamment s'agir de la magnésie (MgO), de Mg(OH)₂, CaO, Ca(OH)₂, BaO, Ba(OH)₂.

Il peut s'agir en particulier d'un catalyseur choisi parmi les oxydes, hydroxydes et sels basiques d'alcalino-terreux et/ou de terres rares ne présentant pas de degré de valence IV et parmi les minéraux en contenant.

On peut notamment mettre en oeuvre les minéraux naturels ou analogues synthétiques qui sont constitués de couches intercalées à base d'oxydes ou d'hydroxydes métalliques, comme l'hydrotalcite. Il peut en particulier s'agir d'une hydrotalcite naturelle ou un analogue synthétique. Ces sels basiques peuvent contenir diverses combinaisons de cations métalliques M²⁺ tels que Mg²⁺, Zn²⁺, Cu^{2+,} Ni²⁺, Te²⁺, Co²⁺ et des cations trivalents de type M³⁺ comme Al³⁺, Cr³⁺, Fe³⁺. Les anions associés à ces cations métalliques peuvent être des halogènes, des anions organiques ou encore des oxanions. A titre représentatif de ces hydrotalcites, on peut en particulier citer celle répondant à la formule [Mg₆Al₂(O₄)₁₆] CO₃.4H₂O.

On peut notamment mettre en oeuvre des oxydes et carbonates de terres rares comme l'Ytterbium et le Lanthane.

Selon un quatrième mode de réalisation ou met en oeuvre un métal alcalin sous forme métallique, par exemple du sodium.

A titre d'exemples de catalyseurs basiques particulièrement utiles, on cite:
- les alcoolate (ou "alkylate") de métal alcalin, notamment le methylate de sodium, l'éthylate de sodium, le tert-butylate de sodium, le methylate de potassium, l'éthylate de potassium, le tert-butylate de potassium
- le sodium métallique,
- l'oxyde de lanthane, ou
- l'oxyde de magnésium.

### Réactions subséquentes

On note qu'on peut mettre en oeuvre une préparation de diesters méthyliques en utilisant du méthanol à titre d'alcool, puis procéder à une trans-estérification avec un alcool plus lourd, comme propanol, isopropanol, alcool benzylique, éthanol, n-butanol, isobutanol, pentanols, cyclohexanol, hexanol, isooctanol, 2-éthyl hexanol et leurs mélanges, pour obtenir un diester plus lourd.

D'autres détails ou avantages de l'invention pourront apparaitre au vu des exemples qui suivent, sans caractère limitatif.

### Exemples

### Exemple 1 - étape a)

On utilise comme produit de départ un mélange de composés dinitriles de composition pondérale suivante:
➢ 86 % en poids de méthylglutaronitrile
➢ 11 % en poids d'éthylsuccinonitrile
➢ 3 % en poids d'adiponitrile.

Sur un lit fixe catalytique composé de 4 ml d'oxyde de titane (anatase) placé entre 2 couches de 5ml de poudre de verre chauffé à 275°C et balayé par un courant d'azote de 3 l/h, on co-injecte à l'aide de 2 pousse seringues 1 ml/h de mélange de dinitriles et 1 ml/h d'eau. En sortie du réacteur, les gaz sont condensés dans une recette placée dans un bain de glace .Apres 6 h de réaction , les produits obtenus sont analysés par chromatographie en phase gazeuse. On obtient alors pour une conversion des dinitriles de 97% un rendement en mélange d'imides de 94%.

### Exemple 2.1 - étape b) avec MgO

Dans un réacteur en inox de 300ml, on introduit 20 g d'un mélange d'imides (produit de l'exemple 1), 175 g de méthanol et 1g de MgO (Prolabo) . On chauffe le mélange réactionnel sous pression autogène à 250°C et on maintient dans ces conditions pendant 6 h. Après refroidissement et filtration du catalyseur on analyse le milieu réactionnel par CPG. On obtient pour une conversion de 90% en imides un rendement en diesters de 67%.

### Exemple 2.2 - étape b) avec de l'oxyde de lanthane

Dans un réacteur en inox de 300ml, on introduit 20 g d'un mélange d'imides (produit de l'exemple 1), 175 g de méthanol et 1g de La2O3 (Rhodia). On chauffe le mélange réactionnel sous pression autogène à 250°C et on maintient dans ces conditions pendant 6 h. Après refroidissement et filtration du catalyseur on analyse le milieu réactionnel par CPG. On obtient pour une conversion de 95% en imides un rendement en diesters de 62%.

### Exemple 2.3 - étape b) avec du méthylate de sodium

Dans un réacteur en inox de 300ml, on introduit 20 g d'un mélange d'imides (produit de l'exemple 1), 175 g de méthanol et 0,5g de méthylate de sodium. On chauffe le mélange réactionnel sous pression autogène à 250°C et on maintient dans ces conditions pendant 6 h. Après refroidissement, on analyse le milieu réactionnel par CPG. On obtient pour une conversion de 92% en imides un rendement en diesters de 65%.

### Exemple 2.4- étape b) avec du tert butylate de potassium

Dans un réacteur en inox de 300ml, on introduit 20 g d'un mélange d'imides (produit de l'exemple 1), 175 g de méthanol et 0,5g de tertbutylate de potassium (Aldrich). On chauffe le mélange réactionnel sous pression autogène à 250°C et on maintient dans ces conditions pendant 6 h. Après refroidissement et filtration du catalyseur on analyse le milieu réactionnel par CPG. On obtient pour une conversion de 89% en imides un rendement en diesters de 67%.

### Exemple 2.5 - étape b) sans catalyseur (Référence)

Dans un réacteur en inox de 300ml, on introduit 20 g d'un mélange d'imides (produit de l'exemple 1) et 175 g de méthanol. On chauffe le mélange réactionnel sous pression autogène à 250°C et on maintient dans ces conditions pendant 6 h. Après refroidissement, on analyse le milieu réactionnel par CPG. On obtient pour une conversion de 85% en imides un rendement en diesters de 65%.

### Exemple 3.1 - Exemple avec huile de Fusel - étape b) avec de l'éthylate de sodium

Dans un réacteur de 300 ml en inox résistant à la pression équipé d'un dispositif de purge continu d'ammoniac, on introduit 25 g d'un mélange d'imides (produit de l'exemple 1), on ajoute 50 g d'huile de fusel de la société WAKO (point d'ébullition 110-130°C -densité 0,810-0,850) et 5% p/p d'éthylate de sodium par rapport à la somme des imides. On ferme le réacteur et on chauffe en agitant le milieu réactionnel à 250°C . Après 4 heures de réaction, la conversion des imides est complète et on obtient un rendement de 90% de diesters. Le milieu réactionnel est filtré pour récupérer le catalyseur et on distille le filtrat pour séparer l'huile de fusel en excès et le mélange de diesters. Le mélange de diesters distille dans la plage 160 -200°C sous 20 mmHg.

### Exemple 3.2 - Exemple avec huile de Fusel - étape b) avec sodium métal

On opère comme dans l'exemple 4 en utilisant 2,5% p/p de sodium métal à la place de l'éthylate de sodium. On obtient alors conversion 100% et rendement en diesters 91%.

## Revendications

1. Procédé de fabrication d'au moins un composé diester comprenant les étapes suivantes:
a) préparation d'un composé imide de forme générale (I) suivante: 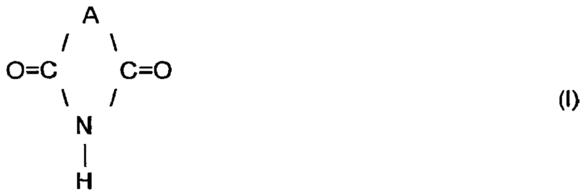 où A représente un radical divalent hydrocarboné comprenant de 2 à 12 atomes de carbones, linéaire ou ramifié,
par hydrolyse en présence d'eau d'au moins un composé dinitrile de formule générale (III) suivante :
NC - A - CN (III)
b) puis réaction entre le composé imide avec au moins un alcool de formule générale (II) suivante:
R - OH (II)
où R représente un radical hydrocarboné pouvant comprendre des hétéroatomes, linéaires ou ramifiés, aliphatique, cycloaliphatique, aromatique ou arylalkyl comprenant de 1 à 20 atomes de carbone,
de manière à obtenir un produit de réaction comprenant au moins un composé diester de formule générale (IV) suivante, et éventuellement des sous-produits de formule(s) différente(s)
R-OOC-A-COO-R (IV)
**caractérisé en ce que**:
- l'étape a) est mise en oeuvre en phase vapeur en présence d'un catalyseur acide solide et
- l'étape b) est mise en oeuvre en présence d'au moins un catalyseur différent de celui mis en oeuvre lors de l'étape a).

2. Procédé selon la revendication 1, **caractérisé en ce que**:
- l'étape b) est mise en oeuvre en présence d'un catalyseur basique, et
- le catalyseur basique est choisi parmi:
- les sels organiques comprenant un anion basique,
- les bases minérales,
- les catalyseurs basiques hétérogènes, et
- les métaux alcalins sous forme métallique.

3. Procédé selon la revendication 2, **caractérisé en ce que** le catalyseur basique comprend:
- un alcoolate de métal alcalin,
- du sodium métallique,
- de l'oxyde de lanthane, ou
- de l'oxyde de magnésium.

4. Procédé selon l'une des revendications 1 à 3, **caractérisé en ce que**:
- l'étape a) est mise en oeuvre en présence d'un catalyseur solide acide, et
- l'étape b) est mise en oeuvre en présence d'un catalyseur basique.

5. Procédé selon l'une des revendications 1 à 4, **caractérisé en ce que** l'étape b) est mise en oeuvre en phase liquide ou vapeur.

6. Procédé selon l'une des revendications 1 à 5, **caractérisé en ce que** l'étape a) est réalisée à une température inférieure à 500 °C de préférence entre 250 et 450 °C.

7. Procédé selon l'une des revendications 1 à 6, **caractérisé en ce que** lors de l'étape a) le rapport molaire entre l'eau et le composé nitrile est compris entre 2 et 20 et préférentiellement compris entre 4 et 8.

8. Procédé selon l'une des revendications 1 à 7, **caractérisé en ce que** lors de l'étape b) le rapport molaire entre l'alcool et le composé imide est compris entre 1 et 30 et préférentiellement compris entre 5 et 20.

9. Procédé selon l'une des revendications 1 à 8, **caractérisé en ce que** le composé dinitrile est choisi dans le groupe comprenant le méthylglutaronitrile, l'éthylsuccinonitrile, l'adiponitrile et leurs mélanges.

10. Procédé selon l'une des revendications 1 à 9, **caractérisé en ce que** l'alcool est choisi dans le groupe comprenant méthanol, propanol, isopropanol, alcool benzylique, éthanol, n-butanol, isobutanol, pentanols, cyclohexanol, hexanol, isooctanol, 2-éthyl hexanol et leurs mélanges.

11. Procédé selon l'une des revendications 1 à 10, **caractérisé en ce que**:
- l'étape a) est mise en oeuvre en présence d'un catalyseur solide acide, et
- le catalyseur solide acide est choisi parmi:
- les oxydes métalliques comme l'alumine, les oxydes de titane, les mélanges silice/alumine et analogues,
- les zéolithes sous forme acide,
- les argiles sous forme acide, et
- les phosphates acides, comme NaH₂PO₄ ou le pyrophosphate de silicium.

12. Procédé selon la revendication 11, **caractérisé en ce que** le catalyseur solide acide comprend du dioxyde de titane anatase.

13. Procédé selon l'une des revendications précédentes, **caractérisé en ce que** l'étape b) est réalisée en phase liquide à une température inférieure à 400 °C de préférence entre 100 et 300 °C, de préférence à une pression de 1 à 100 bars, de préférence la pression autogène.

14. Procédé selon l'une des revendications précédentes, caractérisé en qu'il comprend l'étape suivante c), après l'étape b):
étape c): chauffage du produit de réaction de l'étape b) et distillation de manière à récupérer le composé diester.

15. Procédé selon la revendication 15, **caractérisé en ce que** le produit de réaction de l'étape b) comprend un sous-produit qu'on transforme en imide de formule (I) lors de l'étape c), et qui est re-utilisé pour la mise en oeuvre de l'étape b).

16. Procédé selon l'une des revendications précédentes, **caractérisé en ce que** lors de l'étape b) il se forme de l'ammoniac, qu'on élimine au cours de cette étape.

## Claims

1. Process for the production of at least one diester compound, comprising the following steps:
a) preparation of an imide compound of the following general formula (I): 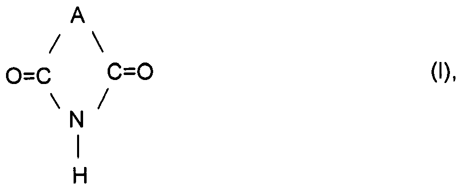 wherein A represents a bivalent, linear or branched, hydrocarbon radical containing from 2 to 12 carbon atoms,
by hydrolysis, in the presence of water, of at least one dinitrile compound of the following general formula (III):
NC - A - CN (III)
b) then reaction of the imide compound with at least one alcohol of the following general formula (II):
R - OH (II),
wherein R represents a linear or branched, aliphatic, cycloaliphatic, aromatic or arylalkyl hydrocarbon radical which may contain hetero atoms, containing from 1 to 20 carbon atoms,
so as to obtain a reaction product comprising at least one diester compound of the following general formula (IV), and optionally sub-products of different formula(e),
R-OOC-A-COO-R (IV),
**characterised in that**:
- step a) is carried out in vapour phase in the presence of a solid acid catalyst, and
- step b) is carried out in the presence of at least one catalyst which is different from that employed in step a).

2. Process according to claim 1, **characterised in that**:
- step b) is carried out in the presence of a basic catalyst, and
- the basic catalyst is selected from:
- organic salts containing a basic anion,
- mineral bases,
- heterogeneous basic catalysts, and
- alkali metals in metallic form.

3. Process according to claim 2, **characterised in that** the basic catalyst comprises:
- an alkali metal alcoholate,
- metallic sodium,
- lanthanum oxide, or
- magnesium oxide.

4. Process according to one of claims 1 to 3, **characterised in that**:
- step a) is carried out in the presence of a solid acid catalyst, and
- step b) is carried out in the presence of a basic catalyst.

5. Process according to one of claims 1 to 4, **characterised in that** step b) is carried out in liquid or vapour phase.

6. Process according to one of claims 1 to 5, **characterised in that** step a) is performed at a temperature less than 500°C, preferably between 250 and 450°C.

7. Process according to one of claims 1 to 6, **characterised in that**, in step a), the molar ratio between the water and the nitrile compound is between 2 and 20, inclusive, and preferably between 4 and 8, inclusive.

8. Process according to one of claims 1 to 7, **characterised in that**, in step b), the molar ratio between the alcohol and the imide compound is between 1 and 30, inclusive, and preferably between 5 and 20, inclusive.

9. Process according to one of claims 1 to 8, **characterised in that** the dinitrile compound is selected from the group comprising methylglutaronitrile, ethylsuccinonitrile, adiponitrile and mixtures thereof.

10. Process according to one of claims 1 to 9, **characterised in that** the alcohol is selected from the group comprising methanol, propanol, isopropanol, benzyl alcohol, ethanol, n-butanol, isobutanol, pentanols, cyclohexanol, hexanol, isooctanol, 2-ethyl hexanol and mixtures thereof.

11. Process according to one of claims 1 to 10, **characterised in that**:
- step a) is carried out in the presence of a solid acid catalyst, and
- the solid acid catalyst is selected from:
- metal oxides such as alumina, titanium oxides, silica/alumina mixtures and similar,
- zeolites in acid form,
- clays in acid form, and
- acid phosphates, such as NaH₂PO₄ or silicon pyrophosphate.

12. Process according to claim 11, **characterised in that** the solid acid catalyst comprises anatase titanium dioxide.

13. Process according to one of the preceding claims, **characterised in that** step b) is performed in liquid phase at a temperature less than 400°C, preferably between 100 and 300°C, preferably at a pressure from 1 to 100 bar, preferably autogenous pressure.

14. Process according to one of the preceding claims, **characterised in that** it comprises the following step c), after step b):
step c): heating the reaction product of step b) and distillation so as to recover the diester compound.

15. Process according to claim 15, **characterised in that** the reaction product of step b) comprises a sub-product which is converted into the imide of formula (I) in step c) and which is re-used in carrying out step b).

16. Process according to one of the preceding claims, **characterised in that**, in step b), ammonia is formed, which is removed in the course of that step.

## Patentansprüche

1. Verfahren zum Herstellen mindestens einer Diesterverbindung, die folgenden Schritte umfassend:
a) Erzeugen einer Imidverbindung der folgenden allgemeinen Formel (I): 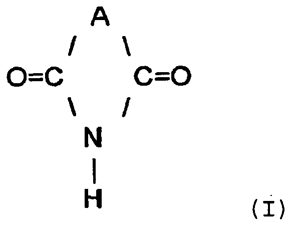 wobei A ein lineares oder verzweigtes zweiwertiges Kohlenwasserstoffradikal darstellt, das 2 bis 12 Kohlenstoffatome besitzt,
mittels Hydrolyse in Anwesenheit von Wasser mindestens einer Dinitrilverbindung der folgenden allgemeinen Formel (III):
NC-A-CN (III)
b) anschließendes Umsetzen der Imidverbindung mit mindestens einem Alkohol der folgenden allgemeinen Formel (II):
R-OH (II)
wobei R ein lineares oder verzweigtes aliphatisches, cycloaliphatisches, aromatisches oder Arylalkyl-Kohlenwasserstoffradikal darstellt, das Heteroatome enthalten kann, das 1 bis 20 Kohlenstoffatome besitzt,
um ein Umsetzungsprodukt zu erhalten, das mindestens eine Diesterverbindung der folgenden allgemeinen Formel (IV) und optional Nebenprodukte mit unterschiedlicher (unterschiedlichen) Formel(n) umfasst,
R-OOC-A-COO-R (IV)
**dadurch gekennzeichnet, dass**:
- Schritt a) in der Dampfphase in Anwesenheit eines festen sauren Katalysators durchgeführt wird, und
- Schritt b) in Anwesenheit mindestens eines Katalysators durchgeführt wird, der sich von dem in Schritt a) eingesetzten unterscheidet.

2. Verfahren gemäß Anspruch 1, **dadurch gekennzeichnet, dass**:
- Schritt b) in Anwesenheit eines basischen Katalysators durchgeführt wird, und
- der basische Katalysator aus
- ein basisches Anion enthaltenden organischen Salzen,
- mineralischen Basen,
- heterogenen basischen Katalysatoren, und
- Alkalimetallen in metallischer Form ausgewählt wird.

3. Verfahren gemäß Anspruch 2, **dadurch gekennzeichnet, dass** der basische Katalysator umfasst:
- ein Alkalimetall-Alkoholat,
- Metallnatrium,
- Lanthanoxid oder
- Magnesiumoxid.

4. Verfahren gemäß einem der Ansprüche 1 bis 3, **dadurch gekennzeichnet, dass**:
- Schritt a) in Anwesenheit eines festen sauren Katalysators durchgeführt wird, und
- Schritt b) in Anwesenheit eines basischen Katalysators durchgeführt wird.

5. Verfahren gemäß einem der Ansprüche 1 bis 4, **dadurch gekennzeichnet, dass** Schritt b) in der Flüssigphase oder in der Dampfphase durchgeführt wird.

6. Verfahren gemäß einem der Ansprüche 1 bis 5, **dadurch gekennzeichnet, dass** Schritt a) bei einer Temperatur von weniger als 500°C, vorzugsweise zwischen 250°C und 450°C durchgeführt wird.

7. Verfahren gemäß einem der Ansprüche 1 bis 6, **dadurch gekennzeichnet, dass** im Schritt a) das molare Verhältnis zwischen dem Wasser und der Nitrilverbindung zwischen 2 und 20 und vorzugsweise zwischen 4 und 8 liegt.

8. Verfahren gemäß einem der Ansprüche 1 bis 7, **dadurch gekennzeichnet, dass** im Schritt b) das molare Verhältnis zwischen dem Alkohol und der Imidverbindung zwischen 1 und 30 und vorzugsweise zwischen 5 und 20 liegt.

9. Verfahren gemäß einem der Ansprüche 1 bis 8, **dadurch gekennzeichnet, dass** die Dinitrilverbindung aus der Methylglutaronitril, Ethylsuccinonitril, Adiponitril und deren Mischungen umfassenden Gruppe ausgewählt wird.

10. Verfahren gemäß einem der Ansprüche 1 bis 9, **dadurch gekennzeichnet, dass** der Alkohol aus der Gruppe umfassend Methanol, Propanol, Isopropanol, Benzylalkohol, Ethanol, n-Butanol, Isobutanol, Pentanole, Cyclohexanol, Hexanol, Isooctanol, 2-Ethylhexanol und deren Mischungen ausgewählt wird.

11. Verfahren gemäß einem der Ansprüche 1 bis 10, **dadurch gekennzeichnet, dass**:
- Schritt a) in Anwesenheit eines festen sauren Katalysators durchgeführt wird, und
- der feste saure Katalysator aus
- Metalloxiden, wie zum Beispiel Aluminiumoxid, Titanoxiden, Siliziumdioxid-/Aluminiumoxid-Gemischen und dergleichen,
- Zeolithen in saurer Form,
- Tonerden in saurer Form, und
- sauren Phosphaten, wie zum Beispiel NaH₂PO₄ oder Siliziumpyrophosphat
ausgewählt wird.

12. Verfahren gemäß Anspruch 11, **dadurch gekennzeichnet, dass** der feste saure Katalysator Anatas-Titandioxid umfasst.

13. Verfahren gemäß einem der vorhergehenden Ansprüche, **dadurch gekennzeichnet, dass** Schritt b) in der Flüssigphase bei einer Temperatur von unter 400°C, vorzugsweise zwischen 100°C und 300°C, vorzugsweise bei einem Druck von 1 bis 100 Bar, vorzugsweise bei autogenem Druck durchgeführt wird.

14. Verfahren gemäß einem der vorhergehenden Ansprüche,
**dadurch gekennzeichnet, dass** es nach Schritt b) den folgenden Schritt c) umfasst:
Schritt c): Erwärmen des Umsetzungsproduktes aus Schritt b) und Destillation, um die Diesterverbindung zurückzugewinnen.

15. Verfahren gemäß Anspruch 15, **dadurch gekennzeichnet, dass** das Umsetzungsprodukt aus Schritt b) ein Nebenprodukt umfasst, das in Schritt c) in ein Imid der Formel (I) umgewandelt wird und zum Durchführen des Schrittes b) wiederverwendet wird.

16. Verfahren gemäß einem der vorhergehenden Ansprüche,
**dadurch gekennzeichnet, dass** sich während des Schrittes b) Ammoniak bildet, der während dieses Schrittes abgetrennt wird.
